(19)

Europäisches
Patentamt
European
Patent Office
Office européen
des brevets

(11) **EP 4 090 239 B1**

(12) **EUROPEAN PATENT SPECIFICATION**

(45) Date of publication and mention
of the grant of the patent:
**20.11.2024  Bulletin 2024/47**

(51) International Patent Classification (IPC):
*A61B 5/06* (2006.01)          *A61B 5/00* (2006.01)
*G06T 3/00* (2024.01)

(21) Application number: **21713143.2**

(22) Date of filing: **15.03.2021**

(52) Cooperative Patent Classification (CPC):
**A61B 5/062; A61B 5/066; A61B 5/6852;**
**A61B 5/7425;** A61B 2560/0238; A61B 2562/0223;
A61B 2562/0233; A61B 2562/0266

(86) International application number:
**PCT/IB2021/052143**

(87) International publication number:
**WO 2021/186330 (23.09.2021 Gazette 2021/38)**

(54) **SYSTEM AND METHOD FOR OPTICAL SENSOR REFERENCE FRAME ALIGNMENT**

SYSTEM UND VERFAHREN ZUR AUSRICHTUNG EINES REFERENZRAHMENS EINES
OPTISCHEN SENSORS

SYSTÈME ET PROCÉDÉ D'ALIGNEMENT DE CADRE DE RÉFÉRENCE DE CAPTEUR OPTIQUE

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**

(30) Priority:  **16.03.2020   US 202062990154 P**

(43) Date of publication of application:
**23.11.2022  Bulletin 2022/47**

(73) Proprietor: **St. Jude Medical International Holding**
**S.à r.l.**
**2449 Luxembourg (LU)**

(72) Inventors:
• **TEGG, Troy**
**Elk River, Minnesota 55330 (US)**
• **DALY, Jacob John**
**Ham Lake, Minnesota 55304 (US)**

(74) Representative: **Mathys & Squire**
**The Shard**
**32 London Bridge Street**
**London SE1 9SG (GB)**

(56) References cited:
**US-A1- 2015 265 368     US-A1- 2016 331 469**
**US-A1- 2019 038 228**

**Description**

**CROSS-REFERENCE TO RELATED APPLICATIONS**

This application claims the benefit of and priority to U.S. provisional application 62/990,154, titled "SYSTEM AND METHOD FOR OPTICAL SENSOR REFERENCE FRAME ALIGNMENT", filed March 16, 2020.

**FIELD**

This disclosure is generally directed to a system for localizing medical devices within a patient, and more particularly to utilizing optical sensors in combination with one or more other localization systems.

**BACKGROUND**

Localization systems are utilized to allow physicians/technicians to visualize the location and/or orientation of a medical device with respect to imaging associated with the patient. For example, electrophysiology catheters are used in a variety of diagnostic, therapeutic, and/or mapping and ablative cardiology procedures to diagnose and/or correct conditions such as atrial arrhythmias, including for example, ectopic atrial tachycardia, atrial fibrillation, and atrial flutter. During a procedure, a catheter or catheter sheath is deployed and manipulated through a patient's vasculature to the intended site, for example, a site within a patient's heart. In order to improve the overall procedure and outcome, it is desirable for an operator to know the position and orientation of the catheter as it is navigated within the body of the patient in order to minimize physical injury to the tissues surrounding the desired organ and ensure that the device reaches its intended target.

Some general methods for localizing medical devices within the patient use fluoropaque markers such as a metallic coil or polymer having a percentage of Barium Sulphate (BaSO4) detected using X-ray fluoroscopy techniques. However, it is desirable to limit patient exposure to X-ray, and therefore it would be beneficial to reduce and/or eliminate reliance of X-ray fluoroscopy techniques. Additional techniques for localizing medical devices include one or more of magnetic, electrical, and/or ultrasound techniques. For example, one type of localization system is an electrical impedance-based system that includes one or more pairs of body surface electrodes (e.g., patches) outside a patient's body, a reference sensor (e.g., another patch) attached to the patient's body, and one or more sensors (e.g., electrodes) attached to the medical device. The pairs can be adjacent, linearly arranged, or associated with respective axes of a reference frame for such a positioning system. The system can determine position and/or orientation by applying a current across pairs of electrodes, measuring respective voltages induced at the device electrodes (i.e., with respect to the reference sensor), and then processing the measured voltages/impedances to determine the location of the device electrodes within the reference frame defined by the external electrodes.

Another system is known as a magnetic field-based system. This type of system generally includes one or more magnetic field generators attached to or placed near the patient bed or other component of the operating environment and one or more magnetic field detection coils coupled with a medical device. The generators provide a controlled low-strength AC magnetic field in the area of interest (i.e., an anatomical region). In response to the magnetic field, the detection coils produce a signal indicative of one or more characteristics of the sensed field. The system then processes these signals to produce one or more position and/or orientation readings associated with the coils (and thus with the medical device). The position and/or orientation readings are typically taken with respect to the field generators, and thus the field generators serve as the de facto "origin" of the reference frame of a magnetic field-based positioning system.

US 2015/265368 A1 relates to systems and methods for navigating a patient anatomy to conduct minimally invasive procedure for dynamically deforming an anatomical passageway model for a display.

**SUMMARY**

The invention is defined by the appended claims. According to one aspect, a medical device comprising may include a proximal end, a distal end, and a shaft extending between the proximal end and the distal end. The medical device may further include a magnetic sensor assembly that may include a magnetic coupler and first and second magnetic sensors, wherein the magnetic coupler is located at the distal end of the medical device and is rigidly affixed to an inner surface of the shaft. The medical device may further include an optical fiber comprised of a plurality of fiber cores extending along a length of the shaft, wherein one or more of the plurality of fiber cores include an optical sensor located at a location along a length of the optical fiber, wherein the optical fiber is rigidly supported within the shaft at a location near the optical sensor.

According to another aspect, a localization system may include a medical device having a proximal end and a distal end, wherein the distal end may include at least a first localization sensor and an optical sensor, wherein the first localization sensor and the optical sensor may be rigidly affixed within the distal end of the medical device. The localization system may further include a computer system configured to receive feedback from the first localization sensor and optical feedback from the optical sensor, wherein the computer system may be utilized to determine the position of the distal end of the medical device within a first reference frame based on the received feedback and may determine the shape of the distal end of the medical device within a second reference frame based on the optical feedback, wherein the computer system may transform the shape of the distal end of the medical device from the second reference frame to the first reference frame based, at least in part, on the position of the distal end of the medical device. An output generated by the computer system may include a position and shape of the distal end of the medical device expressed in the first reference frame.

According to another aspect, a method of localizing a medical device within a patient may include receiving feedback from a first localization sensor and receiving optical feedback from an optical sensor. The method may further include calculating a position of the first localization sensor based on the received feedback, wherein the position is provided with respect to a first reference frame defined by the first localization sensor. The method may further include calculating a shape of the optical sensor based on the optical feedback from the optical sensor, wherein the shape is provided with respect to a second reference frame defined with respect to the optical sensor. The method may further include transforming the shape of the optical sensor from the second reference frame to the first reference frame based on the position of the first localization sensor and stored transformation coefficients. The method may further include displaying the position and shape of the medical device with respect to the first reference frame.

According to another aspect, a method of calibrating an optical sensor with a magnetic localization sensor in a distal end of a medical device may include placing the distal end of a medical device within a magnetic field and placing the distal end of the medical device is a first position, wherein the first position causes a deflection of the optical sensor. The method may further include recording first magnetic position data provided by the magnetic localization sensor and first optical data provided by the optical sensor and storing the recorded data as a first fiducial pair, wherein the first magnetic position data is provided in a magnetic reference frame and the first optical data is provided in an optical reference frame. The method may further include placing the distal end of the medical device in a second position, wherein the second position causes a deflection of the optical sensor and recording second magnetic position data provided by the magnetic localization sensor and second optical data provided by the optical sensor and storing the recorded data as a second fiducial pair, wherein magnetic position data is provided in the magnetic reference frame and the optical data is provided in the optical reference frame. The method may further include calculating a transformation based on the first and second fiducial pairs to transform optical shape data from the optical reference frame to the magnetic reference frame and storing the calculated transformation.

According to another aspect, a medical device may include comprising a proximal end, a distal end, a handle connected to the proximal end, and a shaft extending between the proximal end and the distal end. The medical device may further include first and second magnetic sensors located in the handle and an optical fiber comprised of a plurality of fiber cores extending from the handle to the distal end of the medical device. The optical fiber may be comprised of one or more fiber cores, wherein one or more of the fiber cores includes a plurality of fiber Bragg grating (FBG) sensors located approximately adjacent to one another from the handle to the distal end of the medical device.

## DESCRIPTION OF THE DRAWINGS

FIG. 1 is an isometric view of a localization system using a first medical localization system (e.g., magnetic field-based system) in combination with an optical based system according to some embodiments.

FIG. 2 is an isometric view of the distal end of the catheter having an outer shaft made transparent to illustrate the location of an optical fiber and magnetic sensors within the distal end of the catheter according to some embodiments.

FIG. 3 is an isometric cutaway view of the distal end of the catheter that illustrates a location of an optical sensor and magnetic sensors within a distal end of the catheter according to some embodiments.

FIG. 4 is an isometric cutaway view a multi-core optical fiber according to some embodiments.

FIG. 5 is an isometric view of the optical sensor and magnetic sensors and the reference frame associated with each according to some embodiments.

FIG. 6 is a block diagram of a registration system utilized to register the optical sensor with the first medical localization system according to some embodiments.

FIG. 7 is a flowchart illustrating a method of registering the optical sensor with the first medical localization system according to some embodiments.

FIG. 8 is an isometric view of a handle assembly including magnetic sensors according to some embodiments.

## DETAILED DESCRIPTION

The present disclosure provides a system and method of correlating/displaying shape information received from the optical sensor(s) within the reference frame associated with the first localization system. In this way, the shape of the medical device may be displayed in the reference system of the first localization system. The medical device (e.g., interventional or surgical catheters, introducer, and other elongate medical devices) is equipped with one or more sensors utilized by a first localization system such as magnetic-, electrical impedance-, and/or ultrasound-based systems to localize of the medical device within the body of a patient. In addition, the medical device is equipped with one or more optical sensing technologies, such as fiber Bragg grating (FBG) sensors and/or optical interferometer distal force sensors utilized to detect the shape and/or forces applied to the medical device - or at least a portion of the medical device that includes the optical sensor. In general, optical sensors located on the medical device are configured to receive an optical input via an optical fiber, multi-core fiber, etc. capable of transmitting light signals, wherein information regarding the position, orientation and/or shape of the optical sensor is determined from light reflected by the sensor. The present disclosure provides a system and method of correlating/displaying shape information received from the optical sensor(s) within the reference frame associated with the first localization system.

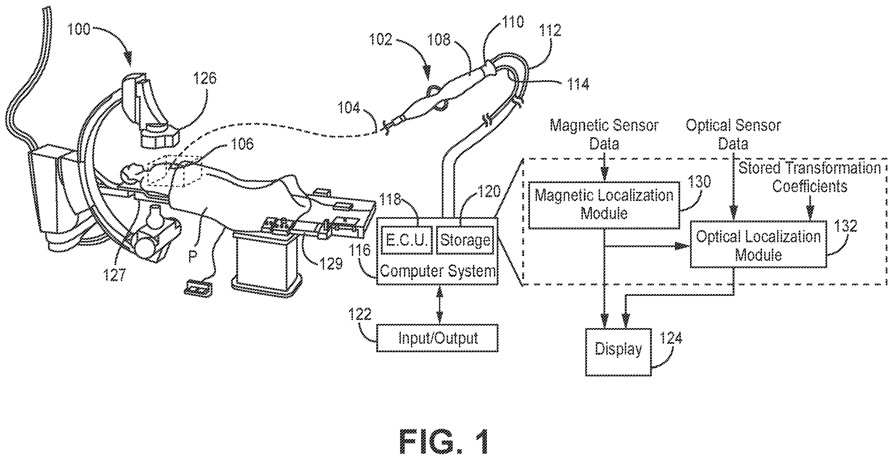

FIG. 1 is an isometric view of a system 100 utilized in performing a medical procedure with respect to a patient P. In the embodiment shown in FIG. 1, the system 100 includes a medical device, such as a catheter 102 shown in FIG. 1, an introducer, or other surgical device including at least a portion of which is located within the body of the patient P. The catheter 102 includes a proximal end 104, a distal end 106, and a handle 108. During a surgical procedure, the distal end 106 is placed within a region of interest within a patient - for example, within the vasculature of the patient - and navigated to a desired location within the body by a physician/technician via controls located on handle 108. As described in more detail below, sensor feedback received from one or more sensors located on the catheter 102 - for example, at the distal end 106 of the catheter 102 - allows computer system 116 to determine the location, orientation, and/or shape of the catheter 102 and to display this information to the physician/technician via display 124. In some embodiments, location, orientation, and/or shape of the catheter 102 is displayed with respect to patient imaging (e.g., MRI imaging, geometries created from mapping catheters, ultrasound catheters, etc.) of the patient. For example, in the embodiment shown in Figure 1, a C-ARM assembly 126 is illustrated, which may be utilized to generate x-ray imaging of a patient P. However, in other embodiments the catheter 102 may be utilized without an external imaging device such as C-Arm 126.

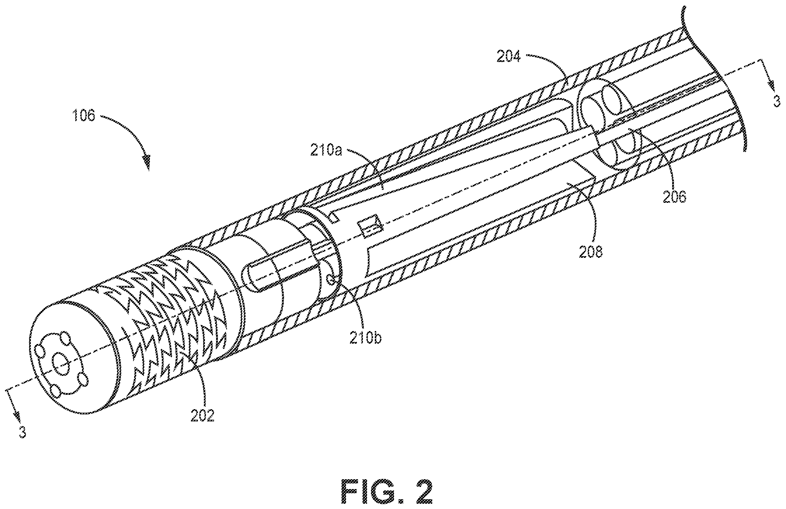

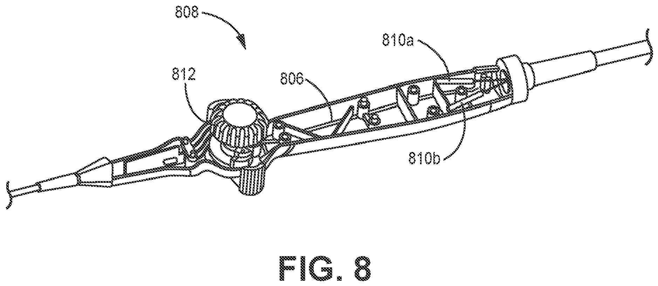

In some embodiments, catheter 102 includes magnetic sensors (210a, 210b shown in FIGS. 2-5) utilized in a magnetic-based localization system to detect the position and/or orientation of the distal end 106 of the catheter 102 within the patient. In some embodiments, the magnetic sensors are located on the distal end 106 of the catheter 102 (for example, as shown in Figures 2, 3 and 5). In other embodiments, the magnetic sensors are located within the handle 108 (for example, in the handle 808 shown in Figure 8). In embodiments utilizing a magnetic-based localization system, a magnetic field must be generated to interact with the magnetic sensors. For example, in some embodiments a magnetic transmitter assembly 127 is mounted to the underside of table 129 to produce the magnetic field required to interact with the magnetic sensors. In embodiments in which the magnetic sensors are located within the handle 108 of the catheter 102, the handle 108 must be operated within the area in which the magnetic field is generated (i.e., in the area adjacent to the patient). In some embodiments, magnetic transmitter assembly 127 generates a low-level magnetic field. One or more magnetic sensors located in the catheter 102 (e.g., distal end 106, handle 108, etc.) interact with the low-level magnetic field and generate in response feedback that can be utilized to determine the position and/or orientation of the magnetic sensors within the region defined by the magnetic field. Feedback generated by the magnetic sensors is provided to the computer system 116 via magnetic sensor cable 114, which interprets the received data and generates a display that allows the physician/technician to visualize the location and orientation of the catheter 102 within imaging of the patient's body via display 124. In some embodiments, an external magnetic field (generated by magnetic transmitter assembly 127) provides a reference for localizing the magnetic sensors within patient imaging and providing this information to display 124. In other embodiments other types of well-known localization systems (e.g., electrical impedance-based systems, ultrasound-based systems, etc.) may be utilized.

In addition, one or more optical sensors provide feedback via fiber core cable 112 to computer system 116. As described in more detail below, optical feedback is utilized to determine position, orientation, shape, and/or temperature of the catheter 102. In particular, the shape information provided by the optical sensor provides information that is not provided by the magnetic-based localization system. However, the optical feedback received from the optical sensors is not referenced to any external field - only to itself. To utilize the shape information provided by the optical sensors, the position, orientation, and/or shape information determined from the optical feedback is converted to the reference frame utilized by the first localization system (e.g. magnetic-based system). As described in more detail below, in some embodiments the optical reference frame associated with the optical sensor is registered with the reference frame utilized by the magnetic sensors (or other sensor type), allowing optical feedback to take advantage of the reference frame defined by the magnetic-based localization system. In this way, position, orientation and/or shape information provided by the optical sensors is transformed (i.e., referenced) to a reference frame capable of displaying position, orientation, shape of the catheter 102 within the context of patient imaging data.

In some embodiments, the computer system 116 includes an electronic control unit (ECU) 118, memory/storage 120, an input/output device 122 and a display 124. Memory/storage 120 stores instructions executable by the processor 118 to implement one or more modules, including a magnetic localization module 130 and an optical localization module 132. In another embodiments, other localization systems may be utilized in place of or in conjunction with the magnetic localization system, including one or more of an electrical impedance-based system, ultrasound system, as well as other well-known localization systems.

Magnetic localization module 130 receives feedback from magnetic sensors located at the distal end 106 of the catheter 102. The feedback provided by the magnetic sensors is a result of the one or more magnetic sensors interacting with the low-level magnetic field generated by the magnetic transmitter assembly 127. In some embodiments, the location of the magnetic sensor is determined within a three-dimensional (3D) reference frame referred to herein as the magnetic reference frame. The output provided by magnetic localization module 130 is a position and/or orientation of the magnetic sensors within the magnetic coordinate frame. Optical sensor data received from the one or more optical sensors located in the catheter 102 are provided to optical localization module 132. In some embodiments, the one or more optical sensors are located at the distal end 106 of catheter 102. In other embodiments, optical sensors may be located at a plurality of locations associated with catheter 102, and may be positioned adjacent or approximately adjacent to one another along the length of the catheter 102 (i.e., from the distal end 106 toward the proximal end 104, in some cases along the entire length to the handle 108). In addition, optical localization module 132 receives input regarding the position of the magnetic sensor within the magnetic coordinate frame and stored transformation coefficients. In some embodiments, a registration process is utilized to register the optical sensor with the magnetic sensor and generate the transformation coefficients utilized to convert positions from the optical reference frame to the magnetic reference frame. Based on the optical sensor data, the position of the magnetic sensor within the magnetic reference frame, and the stored transformation coefficients, the optical localization module 132 generates an output providing the location, orientation and/or shape of the optical sensor within the magnetic reference frame. In this way, the localization output provided with respect to the magnetic sensors and the optical sensors are both expressed within the magnetic reference frame and can be localized with respect to patient imaging data provided to display 124. As described in more detail below, the location of the magnetic sensors may determine the number and location of optical sensors required to express the location, orientation and/or shape of the optical sensor with respect to the magnetic reference frame.

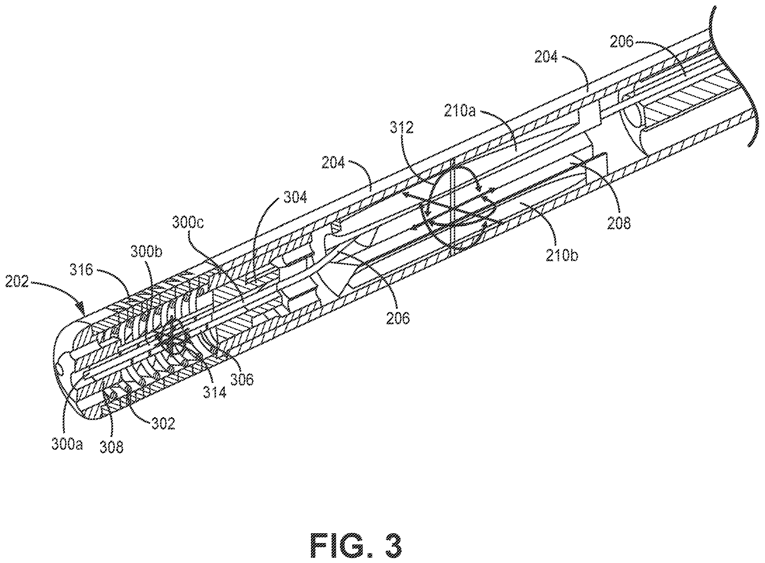

Referring to FIGS. 2-5 and 8, position, orientation, and shape of the medical device is determined within a magnetic reference frame 312 (shown in FIG. 3) based on input received from one or more magnetic sensors (e.g., magnetic sensors 210a, 210b shown in FIG. 2, magnetic sensors 810a, 810b shown in FIG. 8) and input received from one or more optical sensors (e.g., optical sensors 300a, 300b, 300c shown in FIG. 3), such as, for example, a fiber Bragg grating (FBG). As described in more detail below, the magnetic reference frame is based on the interaction of the magnetic sensors 210a, 210b (or 810a, 810b, shown in Figure 8) with an externally created magnetic field. The location and orientation of the magnetic sensors 210a, 210b are therefore known within the magnetic reference frame, and can be localized without additional input. In contrast, the optical reference frame 314 is referenced only to the optical sensor itself, not to any external magnetic field (or in the case of electrical impedance-based localization, to surface electrodes placed on the patient). To remedy the lack of an external reference point with respect to the optical reference frame 314, the optical reference frame 314 is registered with the magnetic reference frame 312. In some embodiments, the output of the registration process is a set of transformation coefficients unique to the medical device registered, wherein the transformation coefficients are utilized to transform feedback received from the one or more optical sensors 300a, 300b, 300c from the optical reference frame 314 to the magnetic reference frame 312. As discussed in more detail below, registering the optical reference frame 314 utilized by the one or more optical sensors 300a, 300b, 300c with the magnetic reference frame 312 includes allowing position/shape information provided by the one or more optical sensors (e.g., FBGs 300a, 300b, 300c) to be displayed within the magnetic reference frame 312 utilized to localize the catheter 102 within patient imaging.

In the embodiment shown in FIG. 3, a plurality of optical sensors 300a, 300b, 300c are located longitudinally along a portion of the multi-core fiber 206, wherein one or more of the cores within the multi-core fiber 206 may include the one or more optical sensors 300a, 300b, 300c . In some embodiments, the one or more optical sensors 300a, 300b, 300c are fiber Bragg grating (FBG) sensors, which is a type of optical sensor capable of detecting applied forces, strains and/or changes in temperature. In some embodiments, a single core of the multi-core fiber 206 includes each of the plurality of optical sensors 300a, 300b, 300c. In addition, the one or more optical sensors may be utilized for a plurality of different functions. For example, one of the optical sensors 300a may be utilized for shape sensing while another of the optical sensors may be utilized for temperature. As described above, in some embodiments additional optical sensors may be positioned along the length of the catheter 102 (from the distal end 106 toward the proximal end 104, in some cases extending to the handle 108), wherein feedback received from the plurality of sensors allows for the shape of the length of the catheter to be calculated. In other embodiments, each of the plurality of optical sensors 300a, 300b, 300c may be utilized for shape sensing, wherein the plurality of optical sensors extend from the distal end 106 of the catheter 102 to the proximal end 104 of the catheter 102, or to the handle 108 of the catheter 102. In this way, the position and/or orientation of the ablation tip 202 and of the shaft 204 may be determined.

As described above, in some embodiments the one or more optical sensors 300a, 300b, 300c are fiber Bragg grating sensors - a type of distributed Bragg reflector that includes a periodic variation in the refractive index of a fiber core. The interface of each change in refractive index results in a reflection of incident light. Most reflections are relatively weak and light at these wavelengths is, for the most part, transmitted through the fiber Bragg grating. However, light at a certain resonance wavelength related to the periodic variation in the refractive index will be reflected by the fiber Bragg grating. The relationship between reflected light and the periodic variation in the refractive index of the fiber core is defined as:

$$\lambda_\beta = 2n_{eff}\Lambda \qquad\qquad (1)$$

wherein $\Lambda$ is the period of the grating, $n_{eff}$ is the fiber core effective index, and $\lambda_\beta$ is the Bragg wavelength at which resonance occurs. In this way, an optical signal transmitted along the core to the fiber Bragg grating will result in the reflection of a resonance wavelength $\lambda_\beta$, which is related to the periodic variation of the fiber Bragg grating. A force, strain and/or change in temperature applied to the fiber grating will result in a change in the period of the grating that in turn causes a change in the wavelength of light reflected. By detecting the shift in wavelengths reflected by a fiber Bragg grating, information regarding force, strain and/or change in temperature applied to the sensor and/or shape (i.e., bending) of the sensor can be detected. In the instant disclosure, the focus is on the detection of force and/or shape applied to the catheter 102, although in some embodiments one or more fiber cores may also be utilized to detect changes in temperature. Force detection may include force applied to the ablative tip along the axis of catheter as well as deflection forces that cause a bending of the catheter 102. Depending on the location of the one or more FBGs, shape information may be collected with respect to specific portions of the catheter 102 (e.g., distal end 106 of the catheter as shown by the placement of one or more FBGs 300a, 300b, 300c) or along the length of the catheter 102. For example, a plurality of FBGs may be positioned along the length of catheter 102 - extending from proximal end 104 or even handle 108 to distal end 106.

When an axial or deflecting force is applied to the fiber grating, both the grating period and the fiber effective index will change accordingly, and hence the Bragg wavelength (e.g., the wavelength of light reflected back) will shift one way or another. By measuring the shift of the Bragg wavelength, the FBGs can be used for force and shape sensing (as well as temperature sensing). One advantage derives from the absolute nature of the information-encoding in measuring the wavelength shift, which renders the sensor independent from fluctuating light power or connector losses. With an applied strain $\varepsilon$ and the ambient temperature change $dT$, the shift of the Bragg wavelength is obtained by taking the differential of Eq. 1 as shown in Eq. 2:

$$\frac{d\lambda}{\lambda_B} = (1 - \rho_e)\varepsilon + (\xi + \alpha)dT \qquad\qquad (2)$$

where $\rho_i = -\frac{1}{n_{eff}}\frac{\partial n_{eff}}{\partial n}$ is the photo-elastic constant, $\rho_e = 0.22$ for pure silica glass, $\alpha = \frac{1}{\Lambda}\frac{\partial \Lambda}{\partial T} \sim 0.5 \times 10^{-6}$ is the coefficient of linear expansion, $\xi = \frac{1}{n_{eff}}\frac{\partial n_{eff}}{\partial T} \sim 7 \times 10^{-6}$ is the thermo-optic coefficient, and dT is the temperature change. For a grating at 1550nm wavelength, the wavelength shifts are typically of order ~1 *pm* / $\mu\varepsilon$ for strain, and 10 *pm/°C* for temperature.

The Young's modulus $E$ is defined as:

$$E = \frac{stress}{strain} = \frac{F/A_0}{\Delta L/L_0} \qquad\qquad (3)$$

where, $F$ is the force, $A_0$ is the area of the fiber cross section, $L_0$ is the fiber length and $\Delta L$ is stressed length due to the applied force. The force can be derived from Eq. (3) as:

$$F = EA_0\varepsilon \qquad\qquad (4)$$

where $\varepsilon = \Delta L/L_0$ is the stain. For a single mode fiber with a diameter of 125 um, the Young's modulus of the glass material is $70 \times 10^9 N/m^2$, then the force with respect to the fiber strain is obtained as:

$$F = 859\varepsilon \ (N) \qquad\qquad (5)$$

When the ambient temperature remains unchanged $dT = 0$, for a pure glass $\rho_e = 0.22$, per Eq. 5 and Eq. 2, the applied force with respect to the shift of the Bragg wavelength is obtained as:

$$F \approx 1101 d\lambda / \lambda_B \qquad\qquad (6)$$

For a resolution of 0.01nm Bragg wavelength shift in 1550nm wavelength band, the force resolution is given by Eq. 6 as 0.7 gram. Per Eq. 4 and 2, the shift of Bragg wavelength with respect to the applied force and temperature change is expressed as

$$\frac{\Delta\lambda}{\lambda_B} = \left(1 - \rho_e\right)\frac{F}{EA_0} + \left(\xi + \alpha\right)\Delta T \qquad\qquad (7)$$

Where $\Delta\lambda$ is the shift of Bragg wavelength, $\Delta T$ is the temperature change, $F$ is the applied force, $E$ is the Young's modulus, $A_0$ is the area of fiber cross section, $\rho_e$ is the photo-elastic constant, $\alpha$ is the coefficient of linear expansion, and f is the thermo-optic coefficient.

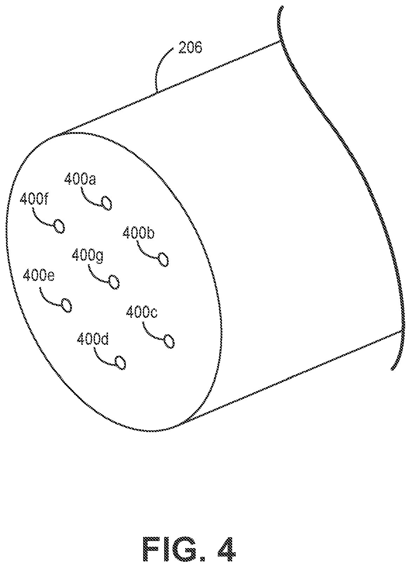

To sense deflection forces in three-dimensions, a plurality of independent optical sensors may be used in one embodiment. For example, with reference to FIG. 4, the multi-core fiber 206 includes a plurality (e.g., seven) individual fiber cores labeled 402a-402g as illustrated, each fiber core including at least one FBG (not shown in FIG. 4). In some embodiments, at least a first plurality of fiber cores (e.g., fiber cores 400a-400f) are positioned equidistantly along the outer circumference of the multi-core fiber 206. A deflection force applied to the multi-core fiber causes some of the optical fibers to lengthen and some of the optical fibers to compress, and thus the feedback provided by some of the FBGs will illustrate a shift of wavelengths corresponding with a compression (i.e., shortening) of the FBG and at least some of the FBGs will illustrate a shift in wavelengths corresponding with a lengthening of the FBG. Based on the feedback, the amount of deflection and shape of the deflection may be determined. Although seven individual fiber cores are shown in Figure 4, in other embodiments fewer individual fiber cores may be required. For example, in some embodiments force and shape sensing is provided by three individual fiber cores located along an outer circumference of a multi-core fiber 206 and spaced equidistantly from one another (e.g., 120° apart). Additionally, more than seven fiber cores in the multi-core fiber 206 are also contemplated.

In some embodiments, the plurality of fiber cores 400a-400f located around an outer circumference are each utilized to detect deflection forces applied to the FBG. In other embodiments, a first plurality of fiber cores located around the periphery (e.g., fiber cores 400a 400c, and 400e) are utilized for shape sensing while a second plurality of fiber cores located around the periphery (e.g., fiber cores 400b, 400d, and 400f) is utilized for axial force sensing. In general, it is desirable that the plurality of cores be spaced equidistantly around the periphery of the multi-core fiber to provide the maximum amount of information with respect to the shape of the sensor. In some embodiments, the central fiber core 400g is also utilized for detecting of forces - including axial and/or deflection forces. In other embodiments, the central fiber core 400g is utilized for temperature compensation/internal strain monitoring. Various other configurations may be utilized to detect deflection forces, axial forces, and/or temperature changes.

As described above, in some embodiments, rather than locating the one or more optical sensors 300a, 300b, 300c (e.g., FBG) at a particular location along the length of the multi-core fiber - for example as part of the ablation tip 202 shown in FIGS. 2 and 3 - one or more of the fiber cores 402a-402g may include a plurality of FBGs located along an axial length of the fiber core. For example, fiber core 400a may include a plurality of FBGs, each defined by a unique grating period. In some embodiments the plurality of FBGs are located adjacent one another (e.g., stacked end-to-end), with little or no gap between the adjacent FBGs. Each FBG provides feedback regarding the forces exerted on the FBG, wherin stacking a plurality of FBGs end-to-end provides shape information for a longer length of the catheter 102. In some embodiments, shape information may be collected with respect to an axial length of the catheter 102 by placing a plurality of FBGs back-to-back. In some embodiments, the pluraltity of FBGs may extend from the distal end 106 of the catheter 102 toward the handle 108 - in some embodiments extending from the distal end 106 of the catheter 102 all the way to the handle 108. In some embodiments, to distiniguish reflections provided by each of the pluraltiy of FBGs connected along the same fiber core, each of the FBGs must be characterized by a different grating period so that reflections received on the same fiber can be definitely assigned to one of the FBGs. In this way, optical sensor 300 provides feedback utilized to determine the shape of the optical sensor 300.

FIG. 3 is a cross-sectional view of the distal end 106 of the catheter 102 taken along line 3-3 shown in FIG. 2. In some embodiments, a magnetic sensor assembly includes magnetic coupler 208 and first and second magnetic sensors 210a, 210b. Magnetic coupler 208 is rigidly affixed to an inner surface of the shaft 204. First and second magnetic sensors 210a, 210b are affixed to magnetic coupler 208, wherein the magnetic coupler 208 retains the magnetic sensors 210a, 210b in fixed locations relative to the shaft 204 as well as with respect to one another. Although not shown in this view, magnetic signals detected by first and second magnetic sensors 210a, 210b, are communicated via magnetic sensor cable 114 (shown in FIG. 1) to computer system 116. Based on the rigid connection of the magnetic sensors 210a, 210b within the distal end 106 of catheter 102 and rigid location of the magnetic sensors 210a, 210b with respect to one another, the location and orientation of the distal end 106 of the catheter 102 is known with respect to a magnetic reference frame 312. In the embodiment shown in FIG. 3, the magnetic reference frame 312 is defined by principle axes (e.g., $mX, mY, mZ$) and orientation about these axes (e.g., yaw, pitch roll) is expressed as $\theta$. Based on feedback provided by the magnetic sensors 210a, 210b, the absolute position and/or orientation of the distal end 106 of the catheter is known within the magnetic reference frame 312.

In some embodiments, optical fiber 206 extends within an interior portion of the outer shaft 204 toward the distal end 106 of catheter 102. In some embodiments, optical fiber 206 is a multi-core fiber including a plurality of fiber cores (as shown in FIG. 4). Optical fiber 206 is rigidly secured within the distal end 106 of the catheter 102 by a fiber tube support member - illustrated here as first fiber tube support 304, second fiber tube support 306 and third fiber tube support 308. In some embodiments, first fiber tube support 304, second fiber tube support 306 and third fiber tube support are integral (i.e., a single tube). In other embodiments, the first, second and third fiber tube supports are separate from one another. In some embodiments, first fiber tube support 304, second fiber tube support 306 and third fiber tube support 308 act to rigidly affix the optical fiber 206 within the distal end 106 of the catheter 102. In the embodiment shown in FIG. 3, first fiber tube support 304, second fiber tube support 306, and third fiber tube support 308 act to support optical fiber 206 in an approximately central location within the distal end 106 of the catheter 102. In other embodiments, optical fiber 206 may be retained within a non-central location of the catheter 102, so long as the position of the optical fiber 206 remains fixed relative to the catheter 102.

Within ablation tip 202, the flex tip wall 316 and spring 302 allow ablation tip 202 to be compressed in an axial direction. In some embodiments, the flex tip wall 316 and spring 302 also allow the ablation tip 202 to deflect in non-axial directions (e.g., to bend). In some embodiments, flex tip wall 316 may be utilized without the inclusion of spring 302. The first, second, and third fiber tube supports 304, 306, and 308 rigidly affix the optical fiber 206 to the catheter 102, forcing the optical fiber 206 and in particular the optical sensor 300 to follow the orientation/shape of the ablation tip 202. In addition, in some embodiments - to ensure rigidity between the optical fiber 206 and the catheter 102 - the optical fiber 206 is bonded to one or more of the first fiber tube support 304, second fiber tube support 306, and/or third fiber tube support 308 to ensure rigid connection of the optical sensor within the shaft 204. As a result, the optical fiber 206 follows the movement and shape of the catheter 102 such that optical feedback received from the optical sensor 300 represents the shape and/or geometry of the catheter 102.

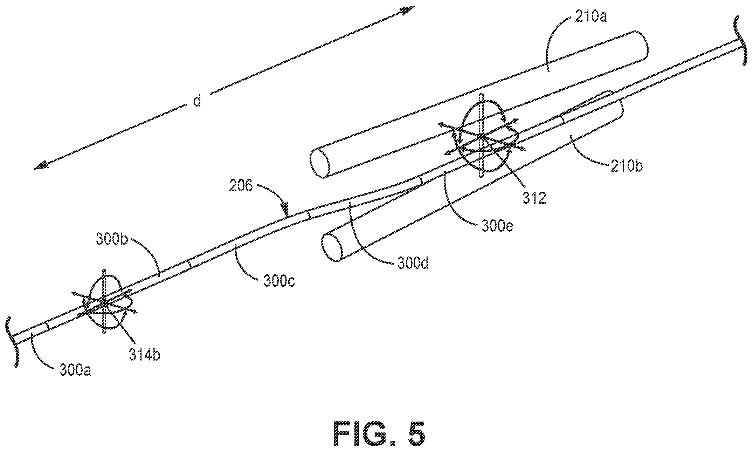

In some embodiments, a plurality of individual optical sensors 300a, 300b, 300c (e.g., FBGs) may be utilized, within one or more associated with each of the plurality of cores. In the embodiment shown in FIG. 3, optical sensor 300a is located at a most distal end of the optical fiber 206, wherein optical sensor 300b is located adjacent to optical sensor 300a, and optical sensor 300c is located adjacent optical sensor 300b. In some embodiments, one or more of the optical sensors 300a, 300b, or 300c may be utilized for shape sensing or force sensing. For example, in some embodiments, optical sensor 300a may be utilized for force sensing, and optical sensors 300b and 300c are utilized for shape sensing. In other embodiments, each of the optical sensors 300a, 300b, and 300c are utilized for shape sensing. In embodiments in which each of the optical sensors 300a, 300b, 300c are located on the same fiber core, each of the plurality of FBGs may be designed to operate at a different wavelength, such that it is capable of providing feedback with respect to sensed strains/forces. In some embodiments, optical sensors 300a-300c may not be constrained to the region within or adjacent to ablation tip 202. For example, in some embodiments a plurality of optical sensors or FBGs may be located end-to-end along one or more of the fiber cores from the ablation tip 202 to magnetic sensors 210a, 210b (for example, in the embodiment shown in FIG. 5, a plurality of optical sensors 300a, 300b, 300c, 300d, and 300e extend from a distal tip of the catheter (300a) to the region adjacent to the magnetic sensors 210a, 210b). In other embodiments, a plurality of optical sensors (e.g., FBG sensors) may be located end-to-end along one or more of the fiber cores from the ablation tip 202 along the length of the catheter 102 to handle 108. With respect to the embodiment shown in FIG. 5, each of the plurality of FBGs 300a-300e - if operating on the same fiber core - may operate at different wavelengths than one another, such that strain/force sensed by each of the plurality of FBGs provides a reflection of light distinguishable from the other FBGs. In this way, feedback provided by the FBGs may be utilized to determine shape information from the magnetic sensors 210a, 210b to the tip of the catheter.

Shape and/or position information retrieved from the optical sensors 300 (provided with respect to the optical reference frame 314) is translated to the magnetic reference frame 312 by registering the one or more optical sensors 300a-300c with the magnetic reference frame 312. In some embodiments, because the magnetic sensors 210a, 210b are rigidly affixed to the distal end 106 of the catheter 102, the magnetic sensors 210a, 210b and therefore the magnetic reference frame 312 is forced to follow the movements of the catheter 102. The position/orientation of the catheter 102 (but not the shape) may therefore be derived from the magnetic sensors 210a, 210b and expressed with respect to the magnetic reference frame 312. Likewise, the one or more optical sensors 300a-300c are rigidly affixed to the distal end 106 of the catheter 012, the optical sensors 300a-300c are also forced to follow the movement of the catheter 102. Registering the one or more optical sensors 300a-300c with respect to the magnetic reference frame 312 allows the magnetic reference frame 312 to be utilized as the reference point for the one or more optical sensors 300a-300c. That is, the position, shape and orientation of the one or more optical sensors 300a-300c is known relative to the position and orientation of the magnetic sensors 210a, 210b.

In the embodiment shown in FIG. 3, the magnetic sensors 210a, 210b are located at the distal end 106 of the catheter 102. In other embodiments, such as the embodiment shown in FIG. 8, the magnetic sensors 810a, 810b are located in the handle 808 of the catheter. In this embodiment, the magnetic reference frame is based on the location of the handle 808. The position and/or orientation of the distal end of the catheter may be determined based on the deployment of a plurality of optical sensors (e.g., FBGs) located along the length of the catheter between the handle 808 and a distal end of the catheter. As described above, each of the adjacent FBG sensors would operate at a different wavelength, allowing each to provide feedback. In particular, referencing the shape data provided by the plurality of optical sensors to the magnetic reference frame defined by the magnetic sensors located in the handle 108 allows for the position and orientation of the distal end 106 of the catheter 102 to be known.

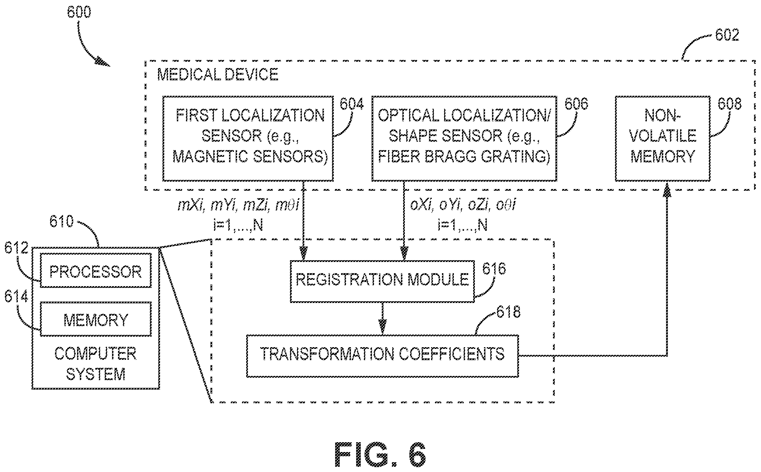

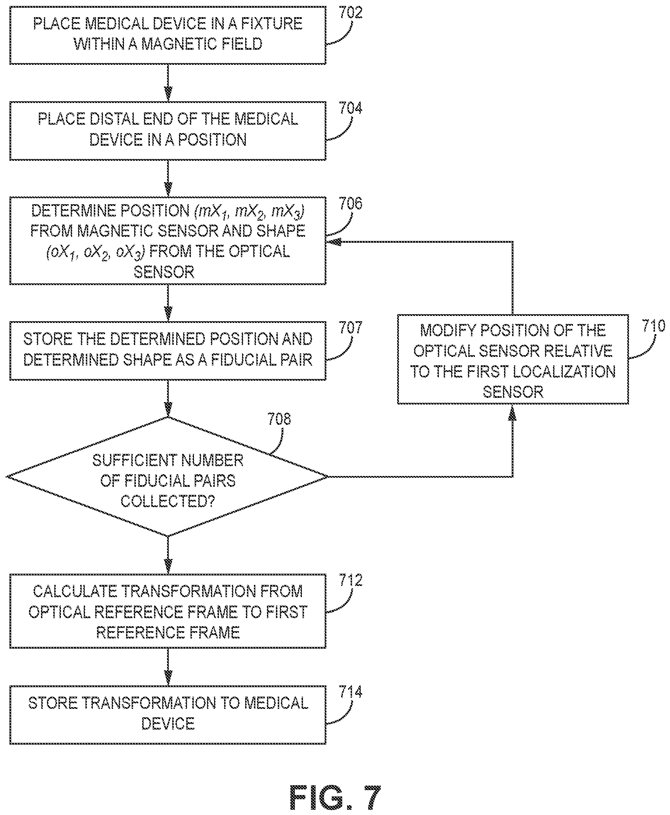

Referring to FIGS. 6 and 7, a registration process is described for correlating the optical reference frame 314 to the magnetic reference frame 312. Although reference is made to the magnetic reference frame 312, in other embodiments other localization systems and corresponding reference frames may be utilized. In some embodiments, the registration process is performed at the time of manufacture. In other embodiments, the registration process if performed prior to each use to re-calibrate the optical reference frame 314 with the magnetic reference frame 312. In particular, FIG. 6 is a block diagram illustrating a system 600 utilized to register the optical sensor 606 according to some embodiments, and FIG. 7 is a flowchart illustrating steps utilized in the registration process according to some embodiments.

The system 600 includes a computer system 610 comprising a processor 612 and memory 614. Memory 614 is configured to store instructions executable by the processor 612 to implement registration module 616. Computer system 610 is configured to receive feedback from medical device 602, which includes magnetic sensors 604, optical sensor 606, and non-volatile memory 608. In some embodiments, magnetic sensor 604 provides feedback that is utilized to determine the position and/or orientation of the medical device 602 in response to the medical device 602 being placed in a magnetic field. Optical sensor 606 likewise generates feedback that is utilized to determine the position, orientation and/or shape of the optical sensor 606. As discussed above with respect to FIGS. 2-5, the feedback received from the magnetic sensors 604 is provided based on the external magnetic field applied and is expressed with respect to the magnetic reference frame $(mX, mY, mZ)$. The feedback received from the optical sensor 606 is provided only with respect to itself and is expressed with respect to an optical reference fame $(oX, oY, oZ)$. Registration module 616 implemented by computer system 610 collects position and orientation data $(mXi, mYi, mZi, m\theta_i)$ from the magnetic sensor 604 and position, orientation, and shape information $(oX_i, oY_i, oZ_i, o\theta_i)$ from the optical sensor 606, and utilizes the collected data to determine transformation data from the optical reference frame to the magnetic reference frame.

With reference to FIG. 7, the process of registering the optical localization/shape sensor 606 within the reference frame utilized by the first localization sensor 604 is illustrated. In some embodiments, the position/shape of the optical sensor 606 is modified relative to the first localization sensor 604 (e.g., magnetic sensors) and feedback from both the sensors are stored as a fiducial pair. A variety of methods of collecting this data is available, although for purposes of this discussion the medical device is held within a fixture that maintains the position of the first localization sensors 604 relatively fixed and the position/shape of the optical sensors 606 are modified relative to the first localization sensors 604. In other embodiments, the optical sensors 606 may be held relatively fixed and the first localization sensors 604 may be modified relative to the optical sensors.

At step 702 the medical device 602 is placed in a fixture (not shown) and the fixture is placed in a magnetic field (assuming the first localization sensor includes magnetic sensors).

At step 704, the portion of the medical device including optical sensors 606 (for example, the distal end 106 of the catheter 102 shown in FIGS. 2-5) is placed in a first position relative to the first localization sensors 604. In subsequent steps, the position of the portion of the medical device 602 including the optical sensors 606 is modified with respect to the first localization sensor 604 in order to collect additional data points describing the relationship between the magnetic reference frame and the optical reference frame. This may be accomplished by maintaining the first localization sensor 604 approximately stationary while the position of the optical sensors 602 is modified. In one embodiment, a force is applied to the portion of the medical device 602 housing the one or more optical sensors 606 (e.g., the distal end 106 of the catheter 102 in the example shown in FIGS. 2-5) to cause a deflection in this portion of the medical device relative to the first localization sensors. For example, in one embodiment the desired force may be generated by attachment of a weight to the distal end of the medical device. In other embodiments, other means may be utilized for deflecting the desired portion of the medical device 602. It is desirable that the medical device 602 be deflected such that optical sensors 606 are compressed/strained to aid in referencing the optical reference frame to the first reference frame

At step 706, feedback received from the first localization sensor 604 is utilized to determine the position and/or orientation ($mX_1$, $mY_1$, $mZ_1$, $m\theta_1$) of the first localization sensors 604 within the first reference frame (e.g. magnetic reference frame). Additionally at step 706, feedback received from the optical sensors 606 is utilized to determine the position, orientation and/or shape ($oX_1$, $oY_1$, $oZ_1$, $o\theta_1$) of the optical sensors 606 within the optical reference frame. As described above with respect to FIGS. 2-5, in some embodiments a plurality of fiber cores are utilized, wherein force applied to the fiber cores is detected via compression/strain of optical sensors (e.g., fiber Bragg grating) located in each of the plurality of fiber cores. The optical reference frame may be centered along one of the plurality of fiber cores, such as central core 400g shown in FIG. 4. In other embodiments, the optical reference frame is centered with respect to one of the plurality of fiber cores located on an outer periphery of the optical conduit (e.g., fiber core 400a).

At step 707, the position data ($mX_1$, $mY_1$, $mZ_1$, $m\theta_1$) and optical-based shape/position data ($oX_1$, $oY_1$, $oZ_1$, $o\theta_1$) are stored as a fiducial pair.

At step 708 a determination is made whether a sufficient number of fiducial pairs have been collected. In some embodiments, this may include comparing a count of fiducial pairs stored with a threshold to determine if additional fiducial pairs are required. In other embodiments, a set number of positions are required and the process continues until fiducial pairs have been collected from each of the required positions. In some embodiments, a predetermined threshold number of fiduciary pairs (collected at sufficiently different positions) is collected. For example, if the medical device is rotated approximately 90 degrees between each measurement, then four fiduciary pairs of data are collected. In other embodiments, the medical device is rotated 45 degrees, and eight fiduciary pairs are collected. In other embodiments, fewer or greater number of fiduciary pairs are collected.

If a sufficient number of fiducial pairs have been collected, then the method proceeds to step 712 wherein the plurality of fiducial pairs are utilized to calculate a transformation from the optical reference frame to the first reference frame (e.g., magnetic reference frame). If a sufficient number of fiducial pairs have not been collected, then the method proceeds to step 710.

At step 710, the position of the portion of the medical device 602 including the optical sensors (e.g., distal end 106 in the embodiment shown in FIGS. 2-5) is modified relative to the location of the first localization sensors 604 (e.g., magnetic sensors). In some embodiments, modification of the position includes rotating the position of the medical device 602 by a known amount (e.g., 90 degrees), which causes rotation of both the optical sensors 606 and the first localization sensor 604. In some embodiments the medical device 602 is rotated within the fixture, while in others the entire fixture holding the medical device 602 is rotated. A force is once again applied to the portion of the medical device 602 including the optical sensors 606 to cause a deflection in the optical sensors 606 relative to the first localization sensors 604. In some embodiments, the same force applied in the previous position is applied in this position, resulting in a deflection of the medical device 606 that is approximately equal in each position. For example, in embodiments in which a weight is attached to the distal end of the medical device 606 to provide the desired deflection force, the same weight applied in the first position is applied in the second position. In other embodiments, the force applied may differ, so long as some force is applied to provide at least some deflection of the optical sensors 606 relative to the first localization sensor 604. In still other embodiments, the portion of the medical device 602 including the optical sensors 606 is moved to a new position relative to the first localization sensor 604.

After modifying the position of the medical device at step 710, at step 706 feedback received from the first localization sensor 604 is utilized to determine a position and/or orientation of the first localization sensor 604 (e.g., $mX_2$, $mY_2$, $mZ_2$, $m\theta_2$) and likewise feedback received from the optical sensors 606 are utilized to determine a position, orientation and/or shape of the optical sensors 606 (e.g., $oX_2$, $oY_2$, $oZ_2$, $o\theta_2$), which are stored as another fiducial pair. The process of modifying the position of the optical sensors relative to the magnetic sensors and measuring the positions of each is continued until a number of fiducial pairs are collected.

At step 712 the transformation required to express position, orientation, and/or shape information collected from the optical sensors 606 in the first reference frame (e.g., $mX$, $mY$, $mZ$) is determined based on the plurality of fiducial pairs. In some embodiments, in addition to the plurality of fiducial pairs, additional information may be utilized in determining the transformation from the optical reference frame (e.g., $oX$, $oY$, $oZ$) to the first reference frame (e.g., $mX$, $mY$, $mZ$). For example, in some embodiments the distance $d$ (shown in FIG. 5) between the magnetic sensors (210a, 210b) and the optical sensors is utilized in the transformation to determine the position of the optical sensor relative to the magnetic reference frame. In this way, position/shape information collected by the optical sensors 606 may be expressed in the first reference frame (e.g., magnetic reference frame).

At step 714, the transformation calculated based on the plurality of fiducial pairs is stored to the medical device 602. In some embodiments, the medical device 602 includes non-volatile memory 608 utilized to store the transformation. During operational use of the medical device 602, the transformation data stored to non-volatile memory 608 may be downloaded or otherwise provided to the computer system (e.g., computer system 116 shown in FIG. 1) to be utilized by the computer system to transform position/shape information provided by the optical sensor 606 to the first reference frame (e.g., magnetic reference frame) to allow the position, orientation, and/or shape of the optical sensor to be displayed accurately within the first reference frame.

With reference to FIG. 8, a handle assembly 808 is illustrated that includes magnetic sensors 810a, 810b according to some embodiments. As described briefly above, in some embodiments the magnetic sensors 810a, 810b may be located within the handle assembly 808 utilized to guide the distal end of the catheter (not shown). In this embodiment, the handle assembly 808 must be placed within the magnetic field - for example within the magnetic field generated by the magnetic transmitter assembly 127 shown in FIG. 1. Based on the feedback provided by the magnetic sensors 810a, 810b, the position and/or orientation of the handle 808 may be determined within the magnetic field (i.e., within the magnetic reference frame). In other embodiments, rather than magnetic sensors, one or more other types of localization systems may be utilized to determine the position of the handle assembly 808.

In some embodiments, fiber core 806 extends from the handle 808 along the length of the shaft from the proximal end to the distal end (as shown in FIG. 1). In some embodiments, the multi-core fiber 806 includes a plurality of fiber cores, wherein one or more of the fiber cores may include one or more optical sensors. For example, in one embodiment fiber Bragg grating (FBG) sensors are located adjacent to one another along the length of the shaft from the handle 808 to the distal end of the catheter. Feedback received from the plurality of FBG sensors allows the shape of the shaft to be calculated from the handle 808 to the distal end. As described above, the position and shape information provided by the plurality of FBGs can be transformed from the reference frame of the FBGs (i.e., the optical reference frame) to the magnetic reference frame of the handle assembly 808. In this way, the position, shape and/or orientation of the catheter from the handle to the distal end may be known and displayed in the magnetic reference frame.

## Claims

1. A localization system (600) comprising:

   a medical device (602) having a proximal end and a distal end, wherein the distal end includes at least a first localization sensor (604) and an optical sensor (606), wherein the first localization sensor (604) and the optical sensor (606) are rigidly affixed within the distal end of the medical device; and
   a computer system (610) configured for:

   receiving feedback from a first localization sensor (604);
   receiving optical feedback from an optical sensor (606);
   calculating a position of the first localization sensor (604) based on the received feedback, wherein the position is provided with respect to a first magnetic reference frame defined by the first localization sensor (604);
   calculating a shape of the optical sensor (606) based on the optical feedback from the optical sensor (606), wherein the shape is provided with respect to a second optical reference frame defined with respect to the optical sensor (606);
   recording first magnetic position data provided by the first localization sensor (604) and first optical data provided by the optical sensor (606);
   storing the recorded data as a first fiducial pair, wherein the first magnetic position data is provided in the magnetic reference frame and the first optical data is provided in the optical reference frame;
   placing the distal end of the medical device (602) in a second position, wherein the second position causes a deflection of the optical sensor (606);
   recording second magnetic position data provided by the first localization sensor (604) and second optical data provided by the optical sensor (606) and storing the recorded data as a second fiducial pair;
   calculating a transformation based on the first and second fiducial pairs to transform the optical shape data form the second optical reference frame to the first magnetic reference frame for transforming the shape of the optical sensor (606) from the second optical reference frame to the first magnetic reference frame; and
   displaying the position and shape of the medical device (602) with respect to the first magnetic reference frame.

2. The localization system of claim 1, wherein the computer system (610) transforms the shape of the distal end of the medical device (602) from the second reference frame to the first reference frame based, additionally, on a transformation correlating the second reference frame to the first reference frame.

3. The localization system of claim 1, wherein the medical device (602) includes non-volatile memory (608) for storing transformation coefficients, wherein the transformation coefficients are uniquely determined during a registration stage to correlate the position of the optical sensor (606) with the position of the first localization sensor (604).

4. The localization system of claim 1, wherein the optical sensor (606) includes one or more fiber Bragg gratings located along a portion of an optical fiber extending along a length of the medical device (602).

5. The localization system of claim 1, wherein the first localization sensor (604) is a magnetic sensor housed within a magnetic coupler rigidly affixed to the distal end of the medical device (602).


**Patentansprüche**

1. Lokalisierungssystem (600), umfassend:

eine medizinische Vorrichtung (602) mit einem proximalen Ende und einem distalen Ende, wobei das distale Ende mindestens einen ersten Lokalisierungssensor (604) und einen optischen Sensor (606) beinhaltet, wobei der erste Lokalisierungssensor (604) und der optische Sensor (606) unbeweglich innerhalb des distalen Endes der medizinischen Vorrichtung befestigt sind; und
ein Computersystem (610), konfiguriert zum:

Empfangen einer Rückmeldung von einem ersten Lokalisierungssensor (604);
Empfangen einer optischen Rückmeldung von einem optischen Sensor (606);
Berechnen einer Position des ersten Lokalisierungssensors (604) auf der Basis der empfangenen Rückmeldung, wobei die Position in Bezug auf einen ersten magnetischen Referenzrahmen, der von dem ersten Lokalisierungssensor (604) definiert wird, bereitgestellt wird;
Berechnen einer Form des optischen Sensors (606) auf der Basis der optischen Rückmeldung von dem optischen Sensor (606), wobei die Form in Bezug auf einen zweiten optischen Referenzrahmen, der in Bezug auf den optischen Sensor (606) definiert ist, bereitgestellt wird;
Aufzeichnen von ersten magnetischen Positionsdaten, die von dem ersten Lokalisierungssensor (604) bereitgestellt werden, und ersten optischen Daten, die von dem optischen Sensor (606) bereitgestellt werden;
Speichern der aufgezeichneten Daten als ein erstes Bezugspaar, wobei die ersten magnetischen Positionsdaten in dem magnetischen Referenzrahmen bereitgestellt werden und die ersten optischen Daten in dem optischen Referenzrahmen bereitgestellt werden;
Platzieren des distalen Endes der medizinischen Vorrichtung (602) in einer zweiten Position, wobei die zweite Position eine Ablenkung des optischen Sensors (606) bewirkt;
Aufzeichnen von zweiten magnetischen Positionsdaten, die von dem ersten Lokalisierungssensor (604) bereitgestellt werden, und zweiten optischen Daten, die von dem optischen Sensor (606) bereitgestellt werden, und Speichern der aufgezeichneten Daten als ein zweites Bezugspaar;
Berechnen einer Umwandlung auf der Basis des ersten und des zweiten Bezugspaars, um die optischen Formdaten von dem zweiten optischen Referenzrahmen in den ersten magnetischen Referenzrahmen zur Umwandlung der Form des optischen Sensors (606) von dem zweiten optischen Referenzrahmen in den ersten magnetischen Referenzrahmen umzuwandeln; und
Anzeigen der Position und der Form der medizinischen Vorrichtung (602) in Bezug auf den ersten magnetischen Referenzrahmen.

2. Lokalisierungssystem nach Anspruch 1, wobei das Computersystem (610) die Form des distalen Endes der medizinischen Vorrichtung (602) von dem zweiten Referenzrahmen in den ersten Referenzrahmen zusätzlich auf der Basis von einer Umwandlung, die den zweiten Referenzrahmen mit dem ersten Referenzrahmen korreliert, umwandelt.

3. Lokalisierungssystem nach Anspruch 1, wobei die medizinische Vorrichtung (602) einen nichtflüchtigen Speicher (608) zum Speichern von Umwandlungskoeffizienten beinhaltet, wobei die Umwandlungskoeffizienten während einer Registrierungsstufe einzigartig bestimmt werden, um die Position des optischen Sensors (606) mit der Position des ersten Lokalisierungssensors (604) zu korrelieren.

4. Lokalisierungssystem nach Anspruch 1, wobei der optische Sensor (606) ein oder mehrere Faser-Bragg-Gitter beinhaltet, die entlang eines Abschnitts eines Lichtwellenleiters angeordnet sind, der sich entlang einer Länge der medizinischen Vorrichtung (602) erstreckt.

5. Lokalisierungssystem nach Anspruch 1, wobei der erste Lokalisierungssensor (604) ein magnetischer Sensor ist, der innerhalb eines magnetischen Kopplers untergebracht ist, der unbeweglich an dem distalen Ende der medizinischen Vorrichtung (602) befestigt ist.

**Revendications**

1. Système de localisation (600) comprenant :

   un dispositif médical (602) ayant une extrémité proximale et une extrémité distale, dans lequel l'extrémité distale inclut au moins un premier capteur de localisation (604) et un capteur optique (606), dans lequel le premier capteur de localisation (604) et le capteur optique (606) sont fixés de manière rigide au sein de l'extrémité distale du dispositif médical ; et
   un système d'ordinateur (610) configuré pour :

      recevoir une rétroaction d'un premier capteur de localisation (604) ;
      recevoir une rétroaction optique d'un capteur optique (606) ;
      calculer une position du premier capteur de localisation (604) sur la base de la rétroaction reçue, dans lequel la position est fournie par rapport à un premier cadre de référence magnétique défini par le premier capteur de localisation (604) ;
      calculer une forme du capteur optique (606) sur la base de la rétroaction optique du capteur optique (606), dans lequel la forme est fournie par rapport à un deuxième cadre de référence optique défini par rapport au capteur optique (606) ;
      enregistrer des premières données de position magnétiques fournies par le premier capteur de localisation (604) et des premières données optiques fournies par le capteur optique (606) ;
      stocker les données enregistrées comme une première paire de repères, dans lequel les premières données de position magnétiques sont fournies dans le cadre de référence magnétique et les premières données optiques sont fournies dans le cadre de référence optique ;
      positionner l'extrémité distale du dispositif médical (602) dans une deuxième position, dans lequel la deuxième position provoque une déviation du capteur optique (606) ;
      enregistrer des deuxièmes données de position magnétiques fournies par le premier capteur de localisation (604) et des deuxièmes données optiques fournies par le capteur optique (606) et stocker les données enregistrées comme une deuxième paire de repères ;
      calculer une transformation sur la base des première et deuxième paires de repères pour transformer les données de forme optique du deuxième cadre de référence optique au premier cadre de référence magnétique destinée à transformer la forme du capteur optique (606) du deuxième cadre de référence optique au premier cadre de référence magnétique ; et
      afficher la position et la forme du dispositif médical (602) par rapport au premier cadre de référence magnétique.

2. Système de localisation selon la revendication 1, dans lequel le système d'ordinateur (610) transforme la forme de l'extrémité distale du dispositif médical (602) du deuxième cadre de référence au premier cadre de référence sur la base, en plus, d'une transformation mettant en corrélation le deuxième cadre de référence et le premier cadre de référence.

3. Système de localisation selon la revendication 1, dans lequel le dispositif médical (602) inclut une mémoire non volatile (608) destinée à stocker des coefficients de transformation, dans lequel les coefficients de transformation sont déterminés uniquement pendant une phase d'enregistrement pour faire corréler la position du capteur optique (606) avec la position du premier capteur de localisation (604).

4. Système de localisation selon la revendication 1, dans lequel le capteur optique (606) inclut un ou plusieurs réseaux de Bragg de fibre situés le long d'une partie d'une fibre optique s'étendant le long d'une longueur du dispositif médical (602).

5. Système de localisation selon la revendication 1, dans lequel le premier capteur de localisation (604) est un capteur magnétique logé au sein d'un coupleur magnétique fixé de manière rigide à l'extrémité distale du dispositif médical (602).

FIG. 1

FIG. 2

EP 4 090 239 B1

EP 4 090 239 B1

**FIG. 3**

EP 4 090 239 B1

FIG. 4

18

FIG. 5

**FIG. 6**

EP 4 090 239 B1

PLACE MEDICAL DEVICE IN A FIXTURE WITHIN A MAGNETIC FIELD — 702

PLACE DISTAL END OF THE MEDICAL DEVICE IN A POSITION — 704

DETERMINE POSITION $(mX_1, mX_2, mX_3)$ FROM MAGNETIC SENSOR AND SHAPE $(oX_1, oX_2, oX_3)$ FROM THE OPTICAL SENSOR — 706

STORE THE DETERMINED POSITION AND DETERMINED SHAPE AS A FIDUCIAL PAIR — 707

SUFFICIENT NUMBER OF FIDUCIAL PAIRS COLLECTED? — 708

MODIFY POSITION OF THE OPTICAL SENSOR RELATIVE TO THE FIRST LOCALIZATION SENSOR — 710

CALCULATE TRANSFORMATION FROM OPTICAL REFERENCE FRAME TO FIRST REFERENCE FRAME — 712

STORE TRANSFORMATION TO MEDICAL DEVICE — 714

FIG. 7

21

FIG. 8

**REFERENCES CITED IN THE DESCRIPTION**

**Patent documents cited in the description**

- US 62990154

- US 2015265368 A1